(19) Europäisches Patentamt
European Patent Office
Office européen des brevets

(11) Veröffentlichungsnummer : **0 345 638 B1**

(12) # EUROPÄISCHE PATENTSCHRIFT

(45) Veröffentlichungstag der Patentschrift :
**16.09.92 Patentblatt 92/38**

(51) Int. Cl.$^5$ : **C07D 231/12,** C08F 20/60, C07D 261/08

(21) Anmeldenummer : **89109914.5**

(22) Anmeldetag : **01.06.89**

(54) **4-[(Meth)Acrylamidomethyl]-pyrazole und -isoxazole, Verfahren zu ihrer Herstellung sowie ihre Verwendung.**

(30) Priorität : **08.06.88 DE 3819456**

(43) Veröffentlichungstag der Anmeldung :
**13.12.89 Patentblatt 89/50**

(45) Bekanntmachung des Hinweises auf die Patenterteilung :
**16.09.92 Patentblatt 92/38**

(84) Benannte Vertragsstaaten :
**BE DE ES FR GB IT NL SE**

(56) Entgegenhaltungen :
**EP-A- 0 188 037**
**CHEMICAL ABSTRACTS, Band 82, Nr. 21, 26. Mai 1975, Seite 629, Zusammenfassung Nr. 140123k, Columbus, Ohio, US**

(73) Patentinhaber : **BASF Aktiengesellschaft**
**Carl-Bosch-Strasse 38**
**W-6700 Ludwigshafen (DE)**

(72) Erfinder : **Bott, Kaspar, Dr.**
**Werderstrasse 57**
**W-6800 Mannheim 1 (DE)**
Erfinder : **Nick, Bernhard, Dr.**
**Sinsheimerstrasse 20**
**W-6700 Ludwigshafen (DE)**
Erfinder : **Schulz, Günther, Dr.**
**Im Roehrich 45**
**W-6702 Bad Duerkheim (DE)**

EP 0 345 638 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 4-[(Meth)Acrylamidomethyl]-pyrazole und -isoxazole.

Die Erfindung betrifft außerdem ein Verfahren zur Herstellung dieser Verbindungen sowie neue Homo- und Copolymerisate, welche unter Verwendung dieser Verbindungen hergestellt worden sind.

Verbindungen, welche eine heterocyclische Gruppe und einen zur Polymerisation befähigten, olefinisch ungesättigten Rest enthalten, sind seit langem bekannt. Charakteristische Beispiele hierfür sind Vinylpyridin, N-Vinylpyrrolidon oder N-Vinylcarbazol. Sie lassen sich leicht zu Homo- und Copolymerisaten umsetzen, welche zwar vielseitig verwendbar sind, aber in einigen Anwendungsgebieten, z.B. auf dem Gebiet der lichtempfindlichen Offset-Druckplatten oder der Oberflächenbeschichtungen, nicht immer alle an sie gestellten Anforderungen erfüllen.

Auf dem Gebiet der Oberflächenbeschichtungen haben die aus der EP-A-188 037 bekannten Verbindungen, in denen eine heterocyclische Gruppe mit einem (Meth)Acrylsäurerest verbunden ist, einen gewissen Fortschritt gebracht. Charakteristische Beispiele für die in der EP-A-188 037 beschriebenen Verbindungen sind

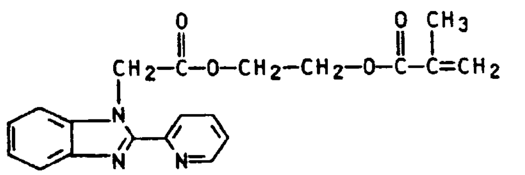
,

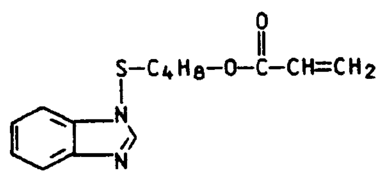

oder

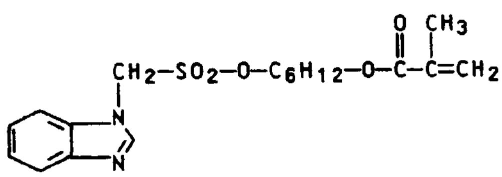
.

In diesen bekannten Verbindungen ist die heterocyclische Gruppe mit dem (Meth)Acryloylrest über eine oder zwei Esterfunktionen verbunden. Die Verwendung der hieraus hergestellten Homo- und Copolymerisate auf dem Gebiet der lichtempfindlichen Offset-Druckplatten geht aus der EP-A-188 037 nicht hervor.

Davon abgesehen, haben diese Verbindungen den Nachteil, daß die darin enthaltenen Esterfunktionen leicht verseifbar sind. Deswegen kommt es in Gegenwart von Wasser, wäßrig-alkalischen Lösungen, Ammoniak oder Aminen zu einer Zersetzung der Verbindungen. Sie sind daher nur für Anwendungszwecke geeignet, bei denen die Gefahr der Zersetzung und damit verbunden des Verlusts der charakteristischen Eigenschaften nicht besteht. Überdies sind bei der Herstellung dieser Verbindungen zahlreiche Syntheseschritte vonnöten, um die Verknüpfung der heterocyclischen Gruppe mit dem (Meth)Acryloylrest zu realisieren, was die Attraktivität dieser Verbindungen noch weiter vermindert.

Aufgabe der vorliegenden Erfindung war es, neue, stabile, einfach herstellbare Verbindungen aufzuzeigen, in denen eine heterocyclische Gruppe mit einem eine radikalisch polymerisierbare, olefinische Doppelbindung enthaltenden Rest verknüpft ist, welche die Nachteile des Standes der Technik nicht mehr aufweisen.

Es wurde nun überraschend gefunden, daß diese Aufgabe durch 4-[(Meth)Acrylamidomethyl]-pyrazole und -isoxazole der nachfolgend näher definierten und beschriebenen Art gelöst wird.

Gegenstand der Erfindung sind dementsprechend 4-[(Meth)Acrylamidomethyl]-pyrazole und -isoxazole der allgemeinen Formel (I)

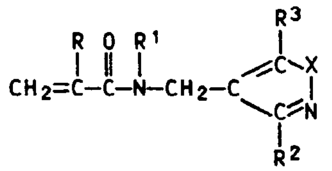 (I),

worin die Reste R, $R^1$, $R^2$ und $R^3$ sowie X, unabhängig voneinander, die folgende Bedeutung haben

R: ein Wasserstoffatom oder eine Methylgruppe, vorzugsweise ein Wasserstoffatom;

$R^1$: ein Wasserstoffatom, eine Methyl- oder eine Ethylgruppe, vorzugsweise ein Wasserstoffatom;

$R^2$, $R^3$: eine Alkylgruppe mit 1 bis 6 C-Atomen oder eine unsubstituierte Arylgruppe mit 6 bis 20 C-Atomen oder 2-, 3- und 4-Methyl-phenyl, 2,4-Dimethyl-phenyl, 4-tert.-Butyl-, 4-Chlor- und 4-Brom-phenyl, 4-Phenyl-phen-1-yl oder 4-(4′-Phenyl-phen-1′-yl)-phen-1-yl, wobei $R^2$ und $R^3$ gleich oder voneinander verschieden sein können;

X: ein Sauerstoffatom oder eine $NR^4$-Gruppe, in der $R^4$ steht für ein Wasserstoffatom oder einen Rest mit der Bedeutung von $R^2$ oder $R^3$.

Als Beispiele für die Reste $R^2$ und $R^3$ in der allgemeinen Formel (I) seien angeführt:

Methyl, Ethyl, n-Propyl, i-Propyl, n-Butyl, sec.-Butyl, tert.-Butyl, Pent-1-, -2- oder -3-yl und Hex-1-, -2- oder -3-yl; weiterhin Phenyl, 2-, 3- und 4-Methyl-phenyl, 2,4-Dimethyl-phenyl, 4-tert.-Butyl-, 4-Chlor- und 4-Brom-phenyl, 4-Phenyl-phen-1-yl (Biphenylyl), 4-(4′-Phenyl-phen-1′-yl)-phen-1-yl (Triphenylyl), 1- und 2-Naphthyl, Phenanthren-7-yl, Anthracen-1-yl, Fluoren-2-yl und Perylen-3-yl.
Von diesen Resten sind Methyl, Ethyl, n-Propyl und Phenyl erfindungsgemäß bevorzugt, wobei die Methyl- und Phenyl-Gruppe ganz besonders bevorzugt sind.

Beispiele für den Rest $R^4$ sind die vorstehend für die Reste $R^2$ und $R^3$ genannten Gruppen. Vorzugsweise stellt $R^4$ ein Wasserstoffatom, eine Methyl-, Ethyl-, n-Propyl- oder Phenylgruppe dar, insbesondere aber ein Wasserstoffatom oder eine Phenylgruppe.

Beispiele bevorzugter erfindungsgemäßer 4-[(Meth)Acrylamidomethyl]-pyrazole und -isoxazole der allgemeinen Formel (I) sind

I-1 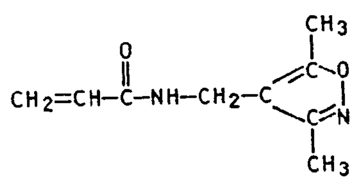 ,

I-2 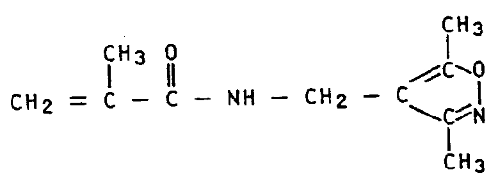 ,

I-3 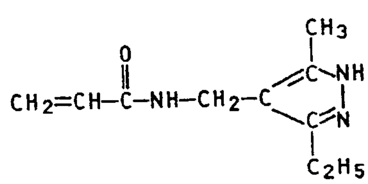 ,

I-4 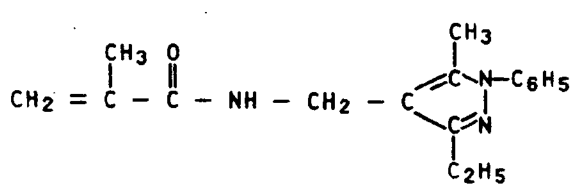 ,

I-5 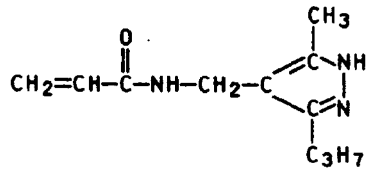 ,

I-6 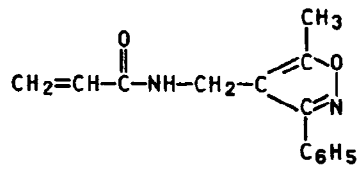 ,

I-7 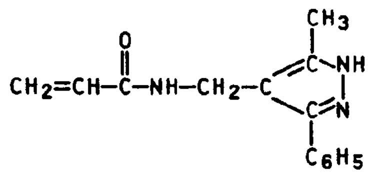 ,

I-8 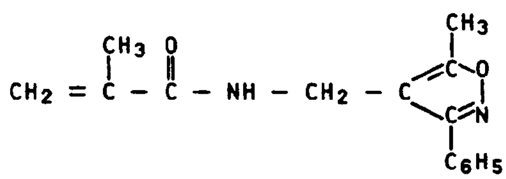 ,

I-9 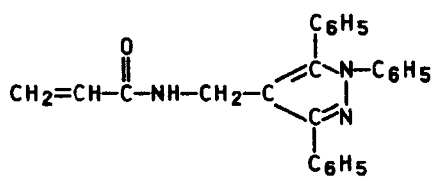 ,

I-10 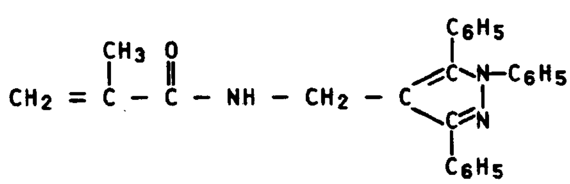

oder

I-11 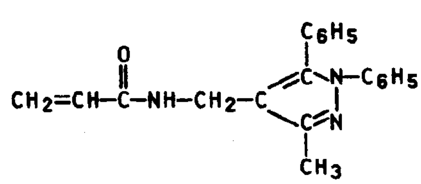 .

Von diesen sind die Verbindungen I-6 (3-Phenyl-4-(acrylamidomethyl)-5-methyl-isoxazol], I-7 (3-Phenyl-4-(acrylamidomethyl)-5-methyl-pyrazol], I-9 [1,3,5-Triphenyl-4-(acrylamidomethyl)-pyrazol] und I-11 (1,5-Diphenyl-3-methyl-4-(acrylamidomethyl)-pyrazol] ganz besonders bevorzugt.

Die Herstellung der erfindungsgemäßen 4-[(Meth)Acrylamidomethyl]-pyrazole und -isoxazole der allgemeinen Formel (I) kann nach den üblichen und bekannten Methoden der präparativen organischen Chemie erfolgen. Von Vorteil ist es indes, die erfindungsgemäßen 4-[(Meth)Acrylamidomethyl]-pyrazole und -isoxazole der allgemeinen Formel (I) nach erfindungsgemäßer Verfahrensweise durch Kondensation eines geeigneten 1,3-Diketons mit (a) einem Hydrazin oder mit (b) einem Hydroxylamin herzustellen.

Dieses erfindungsgemäße Verfahren geht aus von einem 1,3-Diketon der allgemeinen Formel (II)

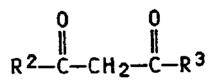

(II),

worin die Reste $R^2$ und $R^3$ gleich oder verschieden voneinander sein können und, unabhängig voneinander, eine Alkylgruppe mit 1 bis 6 C-Atomen oder. 2-, 3- und 4-Methyl-phenyl, 2,4-Dimethyl-phenyl, 4-tert.-Butyl-, 4-Chlor- und 4-Brom-phenyl, 4-Phenyl-phen-1-yl oder 4-(4′-Phenyl-phen-1′-yl)-phen-1-yl oder eine unsubstituierte Arylgruppe mit 6 bis 20 C-Atomen bedeuten Beispiele für die Reste $R^2$ und $R^3$ in der allgemeinen Formel (II) sind die für die Reste $R^2$ und $R^3$ in der allgemeinen Formel (I) genannten Reste. Von diesen Resten werden Methyl, Ethyl, n-Propyl und Phenyl erfindungsgemäß besonders bevorzugt, wobei Methyl und Phenyl ganz besonders bevorzugt sind.

Beispiele für 1,3-Diketone der allgemeinen Formel (II), welche bei dem erfindungsgemäßen Verfahren ganz besonders bevorzugt verwendet werden, sind 2,4-Pentandion (II-1), Benzoylaceton (II-2) und 1,3-Diphenyl-1,3-propandion (II-3).

In erfindungsgemäßer Verfahrensweise werden die 1,3-Diketone der allgemeinen Formel (II) in einer ausreichenden Menge einer starken Säure als Reaktionsmedium bei -5 bis +10°C, insbesondere bei etwa 0°C, gelöst oder dispergiert. Eine geeignete Menge an Säure ist im allgemeinen etwa das 2- bis 20fache des Gewichts des 1,3-Diketons der allgemeinen Formel (II). Geeignete starke Säuren sind u.a. konzentrierte Schwefelsäure, konzentrierte Phosphorsäure oder konzentrierte Trifluormethansulfonsäure, von denen erstere besonders günstig ist.

Zu dieser Lösung oder Dispersion des 1,3-Diketons der allgemeinen Formel (II) fügt man dann die äquimolare Menge eines N-Methylol-(meth)acrylamids der allgemeinen Formel (III) hinzu

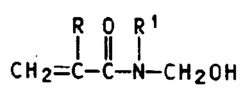

(III),

worin der Rest R ein Wasserstoffatom oder eine Methylgruppe und, unabhängig hiervon, der Rest $R^1$ ein Wasserstoffatom, eine Methyl- oder Ethyl-Gruppe bedeuten. Erfindungsgemäß bevorzugt sind hierbei N-Methylol-(meth)acrylamide der allgemeinen Formel (III), worin $R^1$ für ein Wasserstoffatom steht. Ganz besonders bevorzugt ist das N-Methylol-acrylamid. Die N-Methylol-(meth)acrylamide der allgemeinen Formel (III) können bekanntermaßen in einfacher Weise aus Formaldehyd und dem entsprechenden (Meth)acrylamid hergestellt werden.

Erfindungsgemäß ist es von Vorteil, dem resultierenden Reaktionsgemisch einen üblichen und bekannten thermischen Polymerisationsinhibitor zuzusetzen. Geeignete Polymerisationsinhibitoren sind z.B. Trisnonylphenylphosphit, 2,6-Di-tert.-butyl-p-kresol, Hydrochinonmonomethylether oder polymerisiertes Trimethyldihydrochinon, von denen Hydrochinonmonomethylether besonders vorteilhaft ist. Die Polymerisationsinhibitoren werden dem Reaktionsgemisch in Mengen von 0,001 bis 5 Gew.%, bezogen auf das N-Methylol-(meth)acrylamid der allgemeinen Formel (III), zugegeben.

Das resultierende Reaktionsgemisch aus dem 1,3-Diketon der allgemeinen Formel (II), dem N-Methylol-(meth)acrylamid der allgemeinen Formel (III), der starken Säure sowie ggf. dem Polymerisationsinhibitor wird dann bei Temperaturen von 15 bis 40°C, vorzugsweise 15 bis 30°C und insbesondere 20 bis 25°C, für eine Dauer von 1 bis 48 Stunden, vorzugsweise 2 bis 40 Stunden, und insbesondere 2,5 bis 20 Stunden, gerührt. In dieser Zeit läuft die Kondensationsreaktion zwischen dem 1,3-Diketon der allgemeinen Formel (II) und dem N-Methylol-(meth)acrylamid der allgemeinen Formel (III) zu den betreffenden 2-[(Meth)Acrylamidomethyl]-1,3-diketonen der allgemeinen Formel (IV) ab.

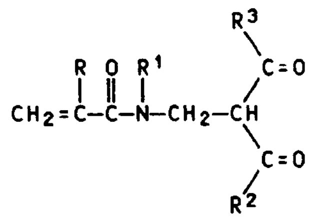

(IV)

In der allgemeinen Formel (IV) kommt den Resten R, $R^1$, $R^2$ und $R^3$ die vorstehend für diese Reste im Zusammenhang mit den allgemeinen Formeln (II) und (III) angegebene Bedeutung zu.

Hiernach gießt man das umgesetzte Reaktionsgemisch auf einen mengenmäßig hohen Überschuß an, vorzugsweise gemahlenem, Eis, wodurch das betreffende 2-[(Meth)Acrylamidomethyl]-1,3-diketon der allgemeinen Formel (IV) ausgefällt wird. Das ausgefällte Produkt sowie in der wäßrigen Phase ggf. noch vorhandenes Produkt werden dann mit einem organischen Lösungsmittel, wie z.B. Toluol, Xylol oder halogenierten Kohlenwasserstoffen, wie Dichlormethan, extrahiert und nach dem Abdampfen des Lösungsmittels aus geeigneten Lösungsmitteln oder Lösungsmittelgemischen umkristallisiert.

Zur Herstellung der erfindungsgemäßen 4-[(Meth)Acrylamidomethyl]-pyrazole der allgemeinen Formel (I) werden die 2-[(Meth)Acrylamidomethyl]-1,3-diketone der allgemeinen Formel (IV) nach dem erfindungsgemäßen Verfahren mit einer, vorzugsweise äquimolaren, Menge eines Hydrazins kondensiert.

Beispiele geeigneter erfindungsgemäß zu verwendender Hydrazine sind Hydraziniumsalze wie Hydraziniumfluorid, -chlorid, -bromid, -hydrogensulfat, -dihydrogenphosphat oder -trifluormethansulfonat, von denen das Hydraziniumhydrogensulfat bevorzugt verwendet wird. Weitere Beispiele geeigneter Hydrazine sind Hydrazinderivate der allgemeinen Formel (V)

$$NH_2\text{-}NH\text{-}R^4 \qquad (V),$$

worin $R^4$ einen der vorstehend beschriebenen Reste $R^2$ oder $R^3$ bezeichnet. Von diesen Hydrazinderivaten der allgemeinen Formel (V) wird Phenylhydrazin bevorzugt verwendet.

Zur Herstellung der erfindungsgemäßen 4-[(Meth)Acrylamidomethyl]-isoxazole der allgemeinen Formel (I) werden die 2-[(Meth)Acrylamidomethyl]-1,3-diketone der allgemeinen Formel (IV) nach dem erfindungsgemäßen Verfahren mit einer, vorzugsweise äquimolaren, Menge an Hydroxylamin, vorzugsweise einem Hydroxylammoniumsalz, kondensiert. Beispiele erfindungsgemäß bevorzugt verwendeter Hydroxylammoniumsalze sind Hydroxylammoniumfluorid, -chlorid, -bromid, -hydrogensulfat, -dihydrogenphosphat oder -trifluormethansulfonat, von denen das Hydroxylammoniumchlorid besonders bevorzugt verwendet wird.

Unabhängig davon, ob man bei dem erfindungsgemäßen Verfahren aus den 2-[(Meth)Acrylamidomethyl]-1,3-diketonen der allgemeinen Formel (IV) die erfindungsgemäßen 4-[(Meth)Acrylamidomethyl]-pyrazole der allgemeinen Formel (I) oder die erfindungsgemäßen 4-[-(Meth)Acrylamidomethyl]- isoxazole der allgemeinen Formel (I) erzeugt, wird die betreffende Kondensation der 1,3-Diketone der allgemeinen Formel (IV) mit den Hydrazinen bzw. den Hydroxylaminen in einem wäßrigen Reaktionsmedium durchgeführt. Hierbei wird unter "wäßrigem Reaktionsmedium" Wasser, welches organische und/oder anorganische Zusatzstoffe gelöst oder dispergiert enthält, verstanden.

Beispiele für geeignete Zusatzstoffe, die in dem wäßrigen Reaktionsmedium enthalten sind, sind u.a. Carbonsäuren, wie Essigsäure, Propionsäure oder Buttersäure, von denen die Essigsäure bevorzugt wird. Weitere Beispiele geeigneter Zusatzstoffe der in Rede stehenden Art sind Carbonsäuresalze, wie Natrium- oder Kaliumacetat, -propionat oder -butyrat, von denen Natriumacetat bevorzugt verwendet wird. Geeignete wäßrige Reaktionsmedien enthalten Wasser und die Zusatzstoffe in einem Gewichtsverhältnis von Wasser zu Zusatzstoffen = 1:5 bis 1:1.

Beispiele für erfindungsgemäß bevorzugt verwendete wäßrige Reaktionsmedien sind

– Wasser plus Essigsäure im Gewichtsverhältnis von 1:5 bis 1:1 oder
– Wasser plus Essigsäure plus Natriumacetat im Gewichtsverhältnis von 1:1:0,05 bis 1:1:0,2.

Die engere Auswahl des für die betreffende Kondensation am besten geeigneten wäßrigen Reaktionsmediums richtet sich vor allem nach der Löslichkeit und/oder Dispergierbarkeit der Reaktanden in diesem wäßrigen Reaktionsmedium und kann anhand einfacher Vorversuche getroffen werden.

Unabhängig davon, in welchem wäßrigen Reaktionsmedium im einzelnen das erfindungsgemäße Verfahren durchgeführt wird, wird zur Durchführung der Kondensationsreaktion entweder so verfahren, daß einer der Reaktanden im wäßrigen Reaktionsmedium vorgelegt und der andere Reaktand portionsweise oder kontinuierlich hinzudosiert wird, oder aber es wird so verfahren, daß beide Reaktanden zugleich in dem betreffenden wäßrigen Reaktionsmedium gelöst und/oder dispergiert werden. Hierbei ist es erfindungsgemäß von Vorteil,

die beiden Reaktanden zugleich zu dem wäßrigen Reaktionsmedium hinzuzugeben und die Kondensation im Eintopfverfahren diskontinuierlich durchzuführen.

Unabhängig davon, in welcher Weise das erfindungsgemäße Verfahren ausgeführt wird, erfolgt die Kondensation zwischen den betreffenden Reaktanden bei der Siedetemperatur des wäßrigen Reaktionsmediums, d.h. unter Rückfluß. Hierbei liegt die Reaktionszeit, nach welcher die Kondensationsreaktion abgelaufen ist, üblicherweise im Bereich von 1 Minute bis 2 Stunden, insbesondere im Bereich von etwa 2 Minuten bis 1 Stunde, und vorzugsweise im Bereich von etwa 3 bis 30 Minuten. Die für die jeweilige Kondensation optimale Reaktionszeit richtet sich nach der Reaktivität der jeweils verwendeten Reaktanden und kann ggf. anhand von Vorversuchen ermittelt werden.

Nach Ablauf der Kondensationsreaktion läßt man das resultierende Reaktionsgemisch auf Zimmertemperatur abkühlen. Hierbei kristallisieren die erfindungsgemäßen 4-[(Meth)Acrylamidomethyl]-pyrazole oder -isoxazole der allgemeinen Formel (I) aus, oder aber sie werden durch Verdünnen des Reaktionsgemisches mit der, bezogen auf das Reaktionsgemisch, zwei- bis zehnfachen Menge an Wasser ausgefällt.

Die auskristallisierten oder ausgefällten Produkte werden in an und für sich üblicher und bekannter Weise, beispielsweise durch Filtration oder Zentrifugieren, isoliert und mit einer geeigneten organischen Flüssigkeit, wie z.B. Diethylether, gewaschen und/oder aus einem geeigneten Lösungsmittel oder Lösungsmittelgemisch, wie z.B. Methanol, Ethanol, Isopropanol oder Ethanol/Isopropanol, umkristallisiert.

Hiernach werden die in erfindungsgemäßer Verfahrensweise erhaltenen 4-[(Meth)Acrylamidomethyl]-pyrazole oder -isoxazole der allgemeinen Formel (I) in aller Regel noch getrocknet. Ihre Charakterisierung kann in üblicher und bekannter Weise erfolgen.

Das erfindungsgemäße Verfahren kann in beliebigen Anlagen oder Apparaturen aus Glas oder Metall ausgeführt werden, wie sie auf dem Gebiet der präparativen organischen Chemie üblich und gebräuchlich sind.

Die erfindungsgemäßen 4-[(Meth)Acrylamidomethyl]-pyrazole und -isoxazole der allgemeinen Formel (I), insbesondere diejenigen, welche nach dem erfindungsgemäßen Verfahren hergestellt worden sind, können aufgrund ihrer besonderen, vorteilhaften Eigenschaften zahlreichen Anwendungszwecken zugeführt werden. So können sie als wertvolle Zwischenprodukte für organische Synthesen dienen. Vor allem können sie als polymerisierbare, insbesondere radikalisch polymerisierbare, und vorzugsweise photopolymerisierbare, Monomere verwendet werden. Sie sind daher besonders geeignet für den Einsatz bei der Herstellung neuartiger, photopolymerisierbarer Dichtungsmassen, Oberflächenschichten, Klebstoffe und flächenförmiger Aufzeichnungsmaterialien, welchen sie ein unerwartet vorteilhaftes anwendungstechnisches Eigenschaftsprofil verleihen.

Besonders vorteilhaft ist der Einsatz der erfindungsgemäßen 4-[(Meth)Acrylamidomethyl]-pyrazole oder -isoxazole der allgemeinen Formel (I) zur Herstellung von Homo- oder Copolymerisaten, wobei die erfindungsgemäßen Pyrazole oder Isoxazole der allgemeinen Formel (I) im Falle der Herstellung von Copolymerisaten miteinander und/oder mit weiteren, an sich bekannten Monomeren copolymerisiert werden können. Hierbei manifestieren sich ihre vorteilhaften Eigenschaften sogar in den Copolymerisaten mit weiteren, an sich bekannten Monomeren, wodurch diese neuen Copolymerisate z.B. ganz vorzüglich als Bindemittel in lichtempfindlichen Offset-Druckplatten geeignet sind.

Ein weiterer wesentlicher Vorteil der erfindungsgemäßen 4-[(Meth)Acrylamidomethyl]-pyrazole und -isoxazole der allgemeinen Formel (I) ist, daß sie nach den von der Polymerchemie her üblichen und bekannten Methoden der thermisch oder photochemisch initiierten radikalischen Polymerisation polymerisiert oder copolymerisiert werden können. So können sie z.B. in Substanz, d.h. in Masse bzw. lösungsmittelfrei, oder in Lösung (co)polymerisiert werden, wobei die üblichen und bekannten Radikalstarter, wie organische Peroxide, Azoverbindungen, sterisch gehinderte Kohlenwasserstoffe ("C-C-Starter"), Redoxinitiatoren oder Photoinitiatoren, angewandt werden können.

Mit besonderem Vorteil können die erfindungsgemäßen 4-[(Meth)Acrylamidomethyl]-pyrazole und -isoxazole der allgemeinen Formel (I) mit den üblichen und bekannten Monomeren aus der Gruppe der

- Vinylaromaten, wie z.B. Styrol, $\alpha$-Methylstyrol oder p-Methylstyrol;
- Nitrile, wie z.B. Acrylnitril oder Methacrylnitril;
- olefinisch ungesättigten Carbonsäuren und deren Anhydride, wie z.B. (Meth)Acrylsäure, (Meth)Acrylsäureanhydrid oder Maleinsäureanhydrid;
- Ester der (Meth)Acrylsäure, wie z.B. Ethyl(meth)acrylat, 2-Hydroxyethyl(meth)acrylat oder Benzylacrylat;
- Ester der Fumar- oder der Maleinsäure, wie z.B. Fumar- oder Maleinsäure-di-n-butyl- oder -di-n-octylester;
- (Meth)Acrylamide, wie z.B. N-Hexyl-(meth)acrylamid;
- Vinylether, wie z.B. Vinylbutylether;
- Vinylester, wie z.B. Vinylacetat oder -propionat;

- Allylether, wie z.B. Allylbutylether;
- Allylester, wie z.B. Itaconsäurebisallylester; oder
- Alkadiene, wie z.B. Butadien oder Isopren;

copolymerisiert werden, wobei diese Aufzählung keinesfalls erschöpfend ist, sondern lediglich charakteristische Beispiele anführt, welche als Hinweis auf weitere geeignete, übliche und bekannte Monomere anzusehen sind.

Unabhängig von ihrem Verwendungszweck und/oder von der Art ihrer Weiterverarbeitung erweisen sich sowohl die erfindungsgemäßen 4-[(Meth)Acrylamidomethyl]-pyrazole und -isoxazole der allgemeinen Formel (I) als auch die hieraus hergestellten erfindungsgemäßen (Co)Polymerisate als ausgesprochen hydrolysestabil. Somit besteht nicht die Gefahr, daß die Verknüpfung zwischen der heterocyclischen Gruppe und dem zur radikalischen Polymerisation befähigten (Meth)Acryloylrest oder dem (Co)Polymerisat durch unerwünschte Nebenreaktionen gespalten wird, was den Verlust der für die erfindungsgemäßen Stoffe charakteristischen, besonders vorteilhaften Eigenschaften zur Folge hätte.

Beispiele

Für die Synthese der erfindungsgemäßen 4-[(Meth)Acrylamidomethyl]-pyrazole und -isoxazole wurden zunächst zwei 2-[(Meth)Acrylamidomethyl]-1,3-diketone hergestellt (Synthesebeispiele I und II).

Synthesebeispiel I

Herstellung und Charakterisierung von 1-Phenyl-2-(acrylamidomethyl)-1,3-butandion

In 500 ml konzentrierte Schwefelsäure wurden bei 0°C der Reihe nach 1,0 g Hydrochinonmonomethylether, 100 g Benzoylaceton und 64,8 g N-Methylol-acrylamid eingetragen. Man ließ das Reaktionsgemisch bei 20°C 4 Stunden lang unter Rühren ausreagieren, wonach es auf 2 kg gemahlenes Eis gegossen wurde. Das hierbei ausgefällte 1-Phenyl-2-(acrylamidomethyl)-1,3-butandion wurde in Dichlormethan gelöst und die resultierende Lösung wurde von der wäßrigen Phase abgetrennt. Anschließend wurde das Dichlormethan verdampft.

Nach der Umkristallisation des Produktes in einem Gemisch aus 3 Vol.-Teilen Cyclohexan und 1 Vol.-Teil Ethanol resultierten 105 g 1-Phenyl-2-(acrylamidomethyl)-1,3-butandion, entsprechend einer Reinausbeute von 70 Gew.%, bezogen auf die Ausgangsprodukte. Das Produkt hatte einen Schmelzpunkt von 125 bis 126°C.

| | | | | |
|---|---|---|---|---|
| Elementaranalyse: | C 68.7 | H 6.0 | O 19.7 | N 5.7 |
| Theorie: | C 68.56 | H 6.16 | O 19.57 | N 5.71 |

Die $^1$H-NMR- und IR-spektroskopischen Messungen bestätigten das Vorliegen der gewünschten Verbindung.

Synthesebeispiel II

Herstellung und Charakterisierung von 1,3-Diphenyl-2-(acrylamidomethyl)-1,3-propandion

Analog zu der in Synthesebeispiel I angegebenen Vorschrift wurde 1,3-Diphenyl-2-(acrylamidomethyl)-1,3-propandion aus 143 g 1,3-Diphenyl-1,3-propandion und 64,8 g N-Methylol-acrylamid hergestellt.

Nach dem Umkristallisieren des Produkts in Methanol wurden 127 g 1,3-Diphenyl-2-(acrylamidomethyl)-1,3-propandion erhalten, was einer Reinausbeute von 65 Gew.% entspricht. Das Produkt hatte einen Schmelzpunkt von 146 bis 148°C.

| | | | | |
|---|---|---|---|---|
| Elementaranalyse: | C 74.1 | H 5.6 | O 15.8 | N 4.5 |
| Theorie: | C 74.25 | H 5.58 | O 15.62 | N 4.56 |

Die $^1$H-NMR- und IR-spektroskopischen Messungen bestätigten das Vorliegen der gewünschten Verbindung.

Beispiel 1

3-Phenyl-4-(acrylamidomethyl)-5-methyl-isoxazol

Eine Mischung aus 34,6 g des nach Synthesebeispiel I hergestellten 1-Phenyl-2-(acrylamidomethyl)-1,3-butandions, 9,84 g Hydroxylammoniumchlorid, 11,6 g Natriumacetat, 180 ml Essigsäure und 180 ml Wasser wurde 5 Minuten lang am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 27,0 g 3-Phenyl-4-(acrylamidomethyl)-5-methyl-isoxazol erhalten, was einer Ausbeute von 79 Gew.% entspricht. Das Produkt hatte einen Schmelzpunkt von 149 bis 151°C.

| | | | | |
|---|---|---|---|---|
| Elementaranalyse: | C 69,4 | H 5,9 | O 13,1 | N 11,6 |
| Theorie: | C 69,41 | H 5,82 | O 13,21 | N 11,56 |

Die $^1$H-NMR- und IR-spektroskopischen Messungen bestätigten das Vorliegen der gewünschten Verbindung.

Beispiel 2

3-Phenyl-4-(acrylamidomethyl)-5-methyl-pyrazol

Eine Mischung aus 29,4 g des nach Synthesebeispiel I hergestellten 1-Phenyl-2-(acrylamidomethyl)-1,3-butandions, 15,6 g Hydraziniumhydrogensulfat, 19,7 g Natriumacetat, 180 ml Essigsäure und 180 ml Wasser wurde während 5 Minuten am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 23,1 g 3-Phenyl-4-(acrylamidomethyl)-5-methyl-pyrazol erhalten, entsprechend einer Ausbeute von 80 Gew.%. Das Produkt hatte einen Schmelzpunkt von 191 bis 193°C.

| | | | |
|---|---|---|---|
| Elementaranalyse: | C 69,4 | H 6,3 | N 17,2 |
| Theorie: | C 69,69 | H 6,27 | N 17,41 |

Die $^1$H-NMR- und IR-spektroskopischen Messungen bestätigten das Vorliegen der gewünschten Verbindung.

Beispiel 3

1,3,5-Triphenyl-4-(acrylamidomethyl)-pyrazol

Ein Gemisch aus 67,4 g des gemäß Synthesebeispiel II hergestellten 1,3-Diphenyl-2-(acrylamidomethyl)-1,3-propandions, 23,8 g Phenylhydrazin, 230 ml Essigsäure und 50 ml Wasser wurde 6 Minuten lang auf 90°C erhitzt. Das nach dem Abkühlen auf Raumtemperatur auskristallisierte Produkt (58,2 g) wurde aus Ethanol umkristallisiert.

Das umkristallisierte Produkt wies einen Schmelzpunkt von 189 bis 191°C auf. Die $^1$H-NMR-spektroskopische Messung bestätigte das Vorliegen von 1,3,5-Triphenyl-4-(acrylamidomethyl)-pyrazol:
$^1$H-NMR (Dimethylsulfoxid; 300 MHz):
$\delta$ = 4,20 (Doublett, 2H, $-CH_2-$)
$\delta$ = 5,60 (Doublett, 1H, H-C=C)
$\delta$ = 6,15 (Doublett, 1H, H-C=C)
$\delta$ = 6,30 (doppeltes Doublett, 1H, H-C=C)
$\delta$ = 7,2-7,8 (Multiplett, 15H, aromatische H)
$\delta$ = 8,60 (Triplett, 1H, NH)

Beispiel 4

1,5-Diphenyl-3-methyl-4-(acrylamidomethyl)-pyrazol

Ein Gemisch aus 29,4 g des gemäß Synthesebeispiel I hergestellten 1-Phenyl-2-(acrylamidomethyl)-1,3-butandions, 12,9 g Phenylhydrazin, 180 ml Essigsäure und 180 ml Wasser wurde 5 Minuten lang am Rückfluß erhitzt und nach dem Abkühlen auf Raumtemperatur mit 600 ml Wasser verdünnt. Die hierbei ausgefällten Kristalle wurden abgesaugt und mit Diethylether nachgewaschen. Auf diese Weise wurden 37,4 g 1,5-Diphenyl-3-methyl-4-(acrylamidomethyl)-pyrazol eines Schmelzpunkts von 157 bis 159°C erhalten. Die $^1$H-NMR-spektroskopische Messung bestätigte das Vorliegen dieses Produkts:
$^1$H-NMR (Dimethylsulfoxid; 300 MHz):
$\delta$ = 2,25 (Singulett, 3H, $CH_3$)
$\delta$ = 4,12 (Doublett, 2H, $-CH_2-$)
$\delta$ = 5,60 (Doublett, 1H, H-C=C)
$\delta$ = 6,12 (Doublett, 1H, H-C=C)
$\delta$ = 6,25 (doppeltes Doublett, 1H, H-C=C)
$\delta$ = 7,10-7,45 (Multiplett, 10H, aromatische H)
$\delta$ = 8,30 (Triplett, 1H, NH).

Beispiel 5

Herstellung eines [3-Phenyl-4-(acrylamidomethyl)-5-methyl-isoxazol]-Copolymerisats

In einen auf 60°C aufgeheizten Vierhalskolben mit Rührer, Innenthermometer, Tropftrichter und Rückflußkühler wurden innerhalb von 4 Stunden

(i) eine Lösung von 7,5 Gew.-Tln. des gemäß Beispiel 1 hergestellten 3-Phenyl-4-(acrylamidomethyl)-5-methyl-isoxazols in 40 Gew.-Tln. Aceton/N-Methylpyrrolidon (Volumenverhältnis 3:1) und

(ii) eine Lösung von 16,3 Gew.-Tln. Acrylnitril, 71,1 Gew.-Tln. Ethylacrylat, 4,3 Gew.-Tln. Methacrylsäure und 0,1 Gew.-Tln. Azo-bis-isobutyronitril in 3 Gew.-Tln. N-Methylpyrrolidon

eindosiert. Hiernach wurden die vereinigten Lösungen unter Rühren eine weitere Stunde am Rückfluß erhitzt.

Anschließend wurde zu der Reaktionslösung innerhalb von einer Stunde

(iii) eine Lösung von 2,3 Gew.-Tln. Methacrylsäure und 0,1 Gew.-Tln. Azo-bis-isobutyronitril in 20 Gew.-Tln. Methanol

hinzudosiert, wonach das resultierende Gemisch eine weitere Stunde am Rückfluß erhitzt wurde. Anschließend wurden

(iv) eine Lösung von 1,2 Gew.-Tln. Methacrylsäure und 0,1 Gew.-Tln. Azo-bis-isobutyronitril in 20 Gew.-Tln. Methanol

sowie

(v) eine Lösung von 2 Gew.-Tln. Azo-bis-isobutyronitril in 80 Gew.-Tln. Methanol

hinzugegeben.

Das resultierende Reaktionsgemisch wurde danach solange am Rückfluß erhitzt, bis die Restmenge an Acrylnitril im nichtflüchtigen Anteil des Reaktionsgemisches kleiner als 0,1 Gew.% war (Bestimmung mittels Gaschromatographie). Hiernach wurde das erhaltene Copolymerisat durch Abdampfen der Lösungsmittel isoliert. Das Copolymerisat hatte einen K-Wert nach Fikentscher von 59 (gemessen an einer 1 gew.%igen Lösung des Copolymerisats in Dimethylformamid) und eine Säurezahl von 58 mg KOH/g.

Beispiel 6

Herstellung eines [3-Phenyl-4-(acrylamidomethyl)-5-methyl-pyrazol]-Copolymerisats

Beispiel 5 wurde wiederholt, nur daß anstelle der dort verwendeten Lösungen (i) und (ii) die Lösungen von

(i) 15 Gew.-Tln. des nach Beispiel 2 erhaltenen 3-Phenyl-4-(acrylamidomethyl)-5-methyl-pyrazols in 45 Gew.-Tln. Methanol und

(ii) 13,6 Gew.-Tln. Acrylnitril, 63,6 Gew.-Tln. Ethylacrylat, 4,3 Gew.-Tln. Methacrylsäure und 0,1 Gew.-Tln. Azo-bis-isobutyronitril in 5 Gew.-Tln. Methanol

verwendet wurden, und daß die vereinigten Lösungen sechs Stunden anstatt einer Stunde am Rückfluß

erhitzt wurden, wonach man, wie in Beispiel 5 angegeben, die übrigen Lösungen (iii) bis (v) zu dem Reaktionsgemisch hinzugab.

Das hierbei resultierende Copolymerisat wies einen K-Wert (nach Fikentscher) von 38,4 und eine Säurezahl von 53 mg KOH/g Copolymerisat auf.

Beispiel 7

Herstellung eines weiteren [3-Phenyl-4-(acrylamidomethyl)-5-methyl-pyrazol]-Copolymerisats

Beispiel 5 wurde wiederholt, nur daß anstelle der dort verwendeten Lösungen (i) bis (v) die folgenden Lösungen verwendet wurden:

(i) 4,125 Gew.-Tln. des gemäß Beispiel 2 erhaltenen 3-Phenyl-4-(acrylamidomethyl)-5-methyl-pyrazols in 18 Gew.-Tln. Methanol,

(ii) 13,6 Gew.-Tln. Acrylnitril, 71,1 Gew.-Tln. Ethylacrylat, 4,3 Gew.-Tln. Methacrylsäure und 0,1 Gew.-Tln. Azo-bis-isobutyronitril in 2 Gew.-Tln. Methanol,

(iii) 2,3 Gew.-Tln. Methacrylsäure und 2,25 Gew.-Tln. des gemäß Beispiel 2 erhaltenen 3-Phenyl-4-(acrylamidomethyl)-5-methyl-pyrazols in 18 Gew.-Tln. Methanol,

(iv) 0,1 Gew.-Tln. Azo-bis-isobutyronitril in 2 Gew.-Tln. Methanol sowie

(v) 1,2 Gew.-Tln. Methacrylsäure und 2,25 Gew.-Tln. des gemäß Beispiel 2 erhaltenen 3-Phenyl-4-(acrylamidomethyl)-5-methyl-pyrazols in 18 Gew.-Tln. Methanol.

Darüber hinaus wurden noch zusätzlich die Lösungen von

(vi) 0,1 Gew.-Tln. Azo-bis-isobutyronitril in 2 Gew.-Tln. Methanol

und hiernach im zeitlichen Abstand von einer Stunde die Lösung von

(vii) 2 Gew.-Tln. Azo-bis-isobutyronitril in 40 Gew.-Tln. Methanol

dem Reaktionsgemisch hinzugefügt.

Das hierbei resultierende Copolymerisat wies einen K-Wert (nach Fikentscher) von 61,3 und eine Säurezahl von 61 mg KOH/g Copolymerisat auf.

Die nach den Beispielen 5, 6 und 7 hergestellten Copolymerisate eigneten sich hervorragend für die Herstellung lichtempfindlicher Schichten von Offset-Druckplatten.

## Patentansprüche

### Patentansprüche für folgende Vertragsstaaten : BE, DE, FR, GB, IT, NL, SE

**1.** 4-[(Meth)Acrylamidomethyl]-pyrazole und -isoxazole der allgemeinen Formel (I)

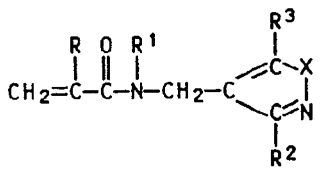

(I),

worin die Reste R, $R^1$, $R^2$ und $R^3$ sowie die Gruppe X, unabhängig voneinander, die folgende Bedeutung haben

R: ein Wasserstoffatom oder eine Methylgruppe;

$R^1$: ein Wasserstoffatom, eine Methyl- oder eine Ethylgruppe;

$R^2$, $R^3$: eine Alkylgruppe mit 1 bis 6 C-Atomen, eine unsubstituierte Arylgruppe mit 6 bis 20 C-Atomen oder 2-, 3- und 4-Methyl-phenyl, 2,4-Dimethyl-phenyl, 4-tert.-Butyl-, 4-Chlor- und 4-Brom-phenyl, 4-Phenyl-phen-1-yl oder 4-(4'-Phenyl-phen-1'-yl)-phen-1-yl, wobei $R^2$ und $R^3$ gleich oder voneinander verschieden sein können;

X: ein Sauerstoffatom oder eine $NR^4$-Gruppe, in der $R^4$ steht für ein Wasserstoffatom oder einen Rest mit der Bedeutung von $R^2$ oder $R^3$.

**2.** 4-[(Meth)Acrylamidomethyl]-pyrazole nach Anspruch 1, dadurch gekennzeichnet, daß in der allgemeinen Formel (I) die Reste R und $R^1$ jeweils ein Wasserstoffatom, die Reste $R^2$ und $R^3$, jeweils für sich, eine Methyl- oder Phenylgruppe und X eine $NR^4$-Gruppe mit $R^4$ = einem Wasserstoffatom oder einer Phenylgruppe bedeuten.

3. 4-[(Meth)Acrylamidomethyl]-isoxazole gemäß Anspruch 1, dadurch gekennzeichnet, daß in der allgemeinen Formel (I) die Reste R und $R^1$ jeweils ein Wasserstoffatom, die Reste $R^2$ und $R^3$, jeweils für sich, eine Methyl- oder Phenylgruppe und X ein Sauerstoffatom bedeuten.

4. Verfahren zur Herstellung von 4-[(Meth)Acrylamidomethyl]-pyrazolen oder -isoxazolen der allgemeinen Formel (I) gemäß einem der Ansprüche 1 bis 3, dadurch gekennzeichnet, daß man in einer ersten Stufe ein 1,3-Diketon der allgemeinen Formel (II)

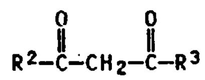 (II),

worin $R^2$ und $R^3$ gleich oder verschieden sein können und unabhängig voneinander eine Alkylgruppe mit 1 bis 6 C-Atomen oder eine unsubstituierte oder substituierte Arylgruppe mit 6 bis 20 C-Atomen bedeuten, mit einem N-Methylol-(meth)acrylamid der allgemeinen Formel (III)

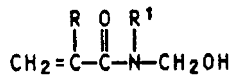 (III),

worin R ein Wasserstoffatom oder eine Methylgruppe und $R^1$ ein Wasserstoffatom, eine Methyl- oder Ethylgruppe bedeuten, in einer starken Säure als Reaktionsmedium kondensiert, wonach man das hierbei erhaltene 2-[(Meth)Acrylamidomethyl]-1,3-diketon der allgemeinen Formel (IV)

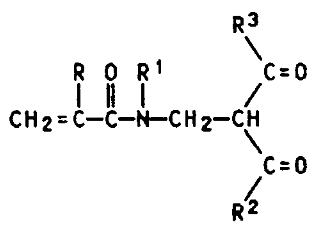 (IV),

worin die Reste R, $R^1$, $R^2$ und $R^3$ die vorstehend für die allgemeinen Formeln (II) und (III) angegebenen Bedeutungen haben, in einer zweiten Stufe mit (a) einem Hydrazin oder (b) einem Hydroxylamin kondensiert.

5. Verfahren nach Anspruch 4, dadurch gekennzeichnet, daß in den allgemeinen Formeln (II), (III) und (IV) die Reste R und $R^1$ jeweils ein Wasserstoffatom und die Reste $R^2$ und $R^3$, jeder für sich, eine Methylgruppe oder eine Phenylgruppe bedeuten.

6. Verfahren nach Anspruch 4 oder 5, dadurch gekennzeichnet, daß man in der zweiten Stufe das 2-[(Meth)Acrylamidomethyl]-1,3-diketon der allgemeinen Formel (IV) mit einem Hydraziniumsalz oder einem Hydrazin der allgemeinen Formel (V)

$$NH_2\text{-}NH\text{-}R^4 \qquad (V),$$

worin $R^4$ ein Wasserstoffatom oder einen Rest mit der Bedeutung von $R^2$ oder $R^3$ bedeutet, kondensiert.

7. Verfahren nach Anspruch 4 oder 5, dadurch gekennzeichnet, daß man in der zweiten Stufe das 2-[(Meth)Acrylamidomethyl]-1,3-diketon der allgemeinen Formel (IV) mit Hydroxylamin oder einem Hydroxylammoniumsalz kondensiert.

8. Verwendung der 4-[(Meth)Acrylamidomethyl]-pyrazole oder -isoxazole der allgemeinen Formel (I) gemäß einem der Ansprüche 1 bis 3 als polymerisierbare, insbesondere photopolymerisierbare, Monomere.

9. Verwendung der 4-[(Meth)Acrylamidomethyl]-pyrazole oder -isoxazole der allgemeinen Formel (I) gemäß einem der Ansprüche 1 bis 3 für die Herstellung photopolymerisierbarer Dichtungsmassen, Oberflächenschichten, Klebstoffe oder flächenförmiger Aufzeichnungsmaterialien.

**10.** Homo- und Copolymerisate der 4-[(Meth)Acrylamidomethyl]-pyrazole der allgemeinen Formel (I) gemäß einem der Ansprüche 1 oder 2.

**11.** Homo- und Copolymerisate der 4-[(Meth)Acrylamidomethyl]-isoxazole der allgemeinen Formel (I) gemäß einem der Ansprüche 1 oder 3.

**Patentansprüche für folgenden Vertragsstaat : ES**

**1.** Verfahren zur Herstellung von 4-[(Meth)Acrylamidomethyl]-pyrazolen oder -isoxazolen der allgemeinen Formel (I)

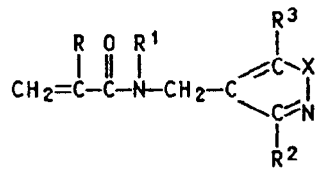

(I),

worin die Reste R, $R^1$, $R^2$ und $R^3$ sowie die Gruppe X, unabhängig voneinander, die folgende Bedeutung haben

R: ein Wasserstoffatom oder eine Methylgruppe;

$R^1$: ein Wasserstoffatom, eine Methyl- oder eine Ethylgruppe;

$R^2$, $R^3$: eine Alkylgruppe mit 1 bis 6 C-Atomen, eine unsubstituierte Arylgruppe mit 6 bis 20 C-Atomen oder 2-, 3- und 4-Methylphenyl, 2,4-Dimethylphenyl, 4-tert-Butyl-, -4-Chlor- und 4-Brom-phenyl, 4-Phenyl-phen-1-yl oder 4-(4′-Phenyl-phen-1′-yl)-phen-1-yl, wobei $R^2$ und $R^3$ gleich oder voneinander verschieden sein können;

X: ein Sauerstoffatom oder eine $NR^4$-Gruppe, in der $R^4$ steht für ein Wasserstoffatom oder einen Rest mit der Bedeutung von $R^2$ oder $R^3$;

dadurch gekennzeichnet, daß man in einer ersten Stufe ein 1,3-Diketon der allgemeinen Formel (II)

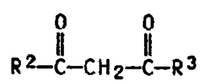

(II),

worin $R^2$ und $R^3$ gleich oder verschieden sein können und unabhängig voneinander eine Alkylgruppe mit 1 bis 6 C-Atomen oder eine unsubstituierte Arylgruppe mit 6 bis 20 C-Atomen oder 2-, 3- und 4-Methylphenyl, 2,4-Dimethylphenyl, 4-tert.-Butyl-, 4-Chlor- und 4-Brom-phenyl, 4-Phenyl-phen-1-yl oder 4-(4′-phenyl-phen-1′-yl)-phen-1-yl bedeuten, mit einem N-Methylol-(meth)acrylamid der allgemeinen Formel (III)

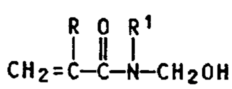

(III),

worin R ein Wasserstoffatom oder eine Methylgruppe und $R^1$ ein Wasserstoffatom, eine Methyl- oder Ethylgruppe bedeuten, in einer starken Säure als Reaktionsmedium kondensiert, wonach man das hierbei erhaltene 2-[(Meth)Acrylamidomethyl]-1,3-diketon der allgemeinen Formel (IV)

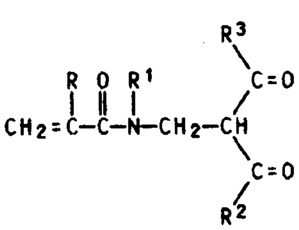

(IV),

worin die Reste R, $R^1$, $R^2$ und $R^3$ die vorstehend für die allgemeinen Formeln (II) und (III) angegebenen Bedeutungen haben, in einer zweiten Stufe mit (a) einem Hydrazin oder (b) einem Hydroxylamin kondensiert.

2. Das Verfahren nach Anspruch 1, dadurch gekennzeichnet, daß in den allgemeinen Formeln (II), (III) und (IV) die Reste R und $R^1$ jeweils ein Wasserstoffatom und die Reste $R^2$ und $R^3$, jeder für sich, eine Methylgruppe oder eine Phenylgruppe bedeuten.

3. Das Verfahren Anspruch 1 oder 2, dadurch gekennzeichnet, daß in der allgemeinen Formel (I) die Reste R und $R^1$ jeweils ein Wasserstoffatom, die Reste $R^2$ und $R^3$, jeweils für sich, eine Methyl- oder Phenylgruppe und X eine $NR^4$-Gruppe mit $R^4$ = einem Wasserstoffatom oder einer Phenylgruppe bedeuten.

4. Das Verfahren nach Anspruch 1 oder 2, dadurch gekennzeichnet, daß in der allgemeinen Formel (I) die Reste R und $R^1$ jeweils ein Wasserstoffatom, die Reste $R^2$ und $R^3$, jeweils für sich, eine Methyl- oder Phenylgruppe und X ein Sauerstoffatom bedeuten.

5. Das Verfahren nach einem der Ansprüche 1 bis 3, dadurch gekennzeichnet, daß man in der zweiten Stufe das 2-[(Meth)Acrylamidomethyl]-1,3-diketon der allgemeinen Formel (IV) mit einem Hydraziniumsalz oder einem Hydrazin der allgemeinen Formel (V)

$$NH_2\text{-}NH\text{-}R^4 \qquad (V),$$

worin $R^4$ ein Wasserstoffatom oder einen Rest mit der Bedeutung von $R^2$ oder $R^3$ bedeutet, kondensiert.

6. Das Verfahren nach einem der Ansprüche 1, 2 oder 4, dadurch gekennzeichnet, daß man in der zweiten Stufe das 2-[(Meth)Acrylamidomethyl]-1,3-diketon der allgemeinen Formel (IV) mit Hydroxylamin oder einem Hydroxylammoniumsalz kondensiert.

7. Verwendung der gemäß einem der Ansprüche 1 bis 3 oder 5 hergestellten 4-[(Meth)Acrylamidomethyl]-pyrazole und der gemäß einem der Ansprüche 1, 2, 4 oder 6 hergestellten 4-[(Meth)Acrylamidomethyl]-isoxazole der allgemeinen Formel (I) als polymerisierbare, insbesondere photopolymerisierbare, Monomere.

8. Verwendung der gemäß einem der Ansprüche 1 bis 3 oder 5 hergestellten 4-[(Meth)Acrylamidomethyl]-pyrazole und der gemäß einem der Ansprüche 1, 2, 4 oder 6 hergestellten 4-[(Meth)Acrylamidomethyl]-isoxazole der allgemeinen Formel (I) für die Herstellung photopolymerisierbarer Dichtungsmassen, Oberflächenschichten, Klebstoffe oder flächenförmiger Aufzeichnungsmaterialien.

9. Verfahren zur Herstellung von Polymerisaten durch Polymerisation von Monomeren, dadurch gekennzeichnet, daß man hierbei als Monomere mindestens ein gemäß einem der Ansprüche 1 bis 3 oder 5 hergestelltes 4-[(Meth)Acrylamidomethyl]-pyrazol und/oder mindestens ein gemäß einem der Ansprüche 1, 2, 4 oder 6 hergestelltes 4-[(Meth)Acrylamidomethyl]-isoxazol verwendet.

10. Das Verfahren zur Herstellung von Polymerisaten nach Anspruch 9, dadurch gekennzeichnet, daß man hierbei als weitere Monomere mindestens ein an sich bekanntes Monomeres verwendet.

## Claims

### Claims for the following Contracting State : ES

1. A process for the preparation of a 4-[(meth)acrylamidomethyl]-pyrazole or -isoxazole of the formula (I)

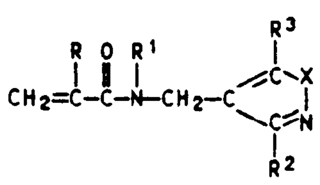

(I)

where R, $R^1$, $R^2$, $R^3$ and X independently of one another have the following meanings: R is hydrogen or

methyl, $R^1$ is hydrogen, methyl or ethyl, $R^2$ and $R^3$ may be identical or different and are each alkyl of 1 to 6 carbon atoms or unsubstituted aryl of 6 to 20 carbon atoms or 2-, 3- or 4-methylphenyl, 2,4 -dimethylphenyl, 4-tert-butyl-, 4-chloro- or 4-bromophenyl, 4-phenylphen-1-yl or 4-(4'-phenylphen-1'-yl)-phen-1-yl and X is oxygen or an $NR^4$ group, where $R^4$ is hydrogen or a radical having the meanings of $R^2$ or $R^3$ wherein, in a first step, a 1,3-diketone of the formula (II)

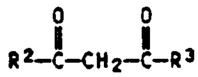

(II)

where $R^2$ and $R^3$ may be identical or different and independently of one another are each alkyl of 1 to 6 carbon atoms or unsubstituted aryl of 6 to 20 carbon atoms or 2-, 3- or 4-methylphenyl, 2,4-dimethylphenyl, 4-tert-butyl-, 4-chloro- or 4-bromophenyl, 4-phenylphen-1-yl or 4-(4'-phenylphen-1'-yl)-phen-1-yl, is condensed with an N-methylol(meth)acrylamide of the formula (III)

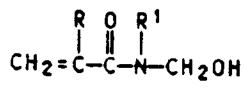

(III)

where R is hydrogen or methyl and $R^1$ is hydrogen, methyl or ethyl, in a strong acid as reaction medium, whereupon the 2-[(meth)acrylamidomethyl]-1,3-diketone of the formula (IV) obtained here

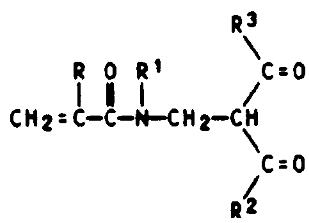

(IV)

where R, $R^1$, $R^2$ and $R^3$ have the meanings stated above for the formulae (II) and (III), is condensed in a second step with (a) a hydrazine or (b) a hydroxylamine.

2. The process as claimed in claim 1, wherein, in the formulae (II), (III) and (IV), R and $R^1$ are each hydrogen and $R^2$ and $R^3$ are each methyl or phenyl.

3. The process as claimed in claim 1 or 2, wherein, in the formula (I), R and $R^1$ are each hydrogen, $R^2$ and $R^3$ are each methyl or phenyl and X is $NR^4$ where $R^4$ is hydrogen or phenyl.

4. The process as claimed in claim 1 or 2, wherein, in the formula (I), R and $R^1$ are each hydrogen, $R^2$ and $R^3$ are each methyl or phenyl and X is oxygen.

5. The process as claimed in any of claims 1 to 3, wherein, in the second step, a 2-[(meth)acrylamidomethyl]-1,3-diketone of the formula (IV) is condensed with a hydrazinium salt or a hydrazine of the formula (V)

$$NH_2\text{-}NH\text{-}R^4 \qquad (V)$$

where $R^4$ is hydrogen or a radical having the meanings of $R^2$ or $R^3$.

6. The process as claimed in any of claims 1, 2 or 4, wherein, in the second step, a 2-[(meth)acrylamidomethyl]-1,3-diketone of the formula (IV) is condensed with hydroxylamine or a hydroxylammonium salt.

7. Use of a 4-[(meth)acrylamidomethyl]-pyrazole prepared as claimed in any of claims 1 to 3 or 5 or of a 4-[(meth)acrylamidomethyl]-isoxazole prepared as claimed in any of claims 1, 2, 4 or 6, of the formula (I), as a polymerizable, in particular photopolymerizable, monomer.

8. Use of a 4-[(meth)acrylamidomethyl]-pyrazole prepared as claimed in any of claims 1 to 3 or 5 or of a 4-[(meth)acrylamidomethyl]-isoxazole prepared as claimed in any of claims 1, 2, 4 or 6, of the formula (I), for the preparation of photopolymerizable sealing compounds, surface layers, adhesives or sheet-like recording materials.

**9.** A process for the preparation of a polymer by polymerization of monomers, wherein at least one 4-[(meth)acrylamidomethyl]-pyrazole prepared as claimed in any of claims 1 to 3 or 5 and/or at least one 4-[(meth)acrylamidomethyl]-isoxazole prepared as claimed in any of claims 1, 2, 4 or 6 are used here as monomers.

**10.** The process for the preparation of a polymer as claimed in claim 9, wherein at least one conventional monomer is used here as a further monomer.

**Claims for the following Contracting States : BE, DE, FR, GB, IT, NL, SE**

**1.** A 4-[(meth)acrylamidomethyl]-pyrazole or -isoxazole of the formula (I)

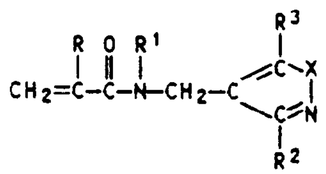

(I)

where R, $R^1$, $R^2$, $R^3$ and X independently of one another have the following meanings: R is hydrogen or methyl, $R^1$ is hydrogen, methyl or ethyl, $R^2$ and $R^3$ may be identical or different and are each alkyl of 1 to 6 carbon atoms or unsubstituted aryl of 6 to 20 carbon atoms or 2-, 3- or 4-methylphenyl, 2,4-dimethylphenyl, 4-tert-butyl-, 4-chloro- or 4-bromophenyl, 4-phenylphen-1-yl or 4-(4′-phenylphen-1′-yl)-phen-1-yl and X is oxygen or an $NR^4$ group, where $R^4$ is hydrogen or a radical having the meanings of $R^2$ or $R^3$.

**2.** A 4-[(meth)acrylamidomethyl]-pyrazole as claimed in claim 1, wherein, in the formula (I), R and $R^1$ are each hydrogen, $R^2$ and $R^3$ are each methyl or phenyl and X is $NR^4$ where $R^4$ is hydrogen or phenyl.

**3.** A 4-[(meth)acrylamidomethyl]-isoxazole as claimed in claim 1, wherein, in the formula (I), R and $R^1$ are each hydrogen, $R^2$ and $R^3$ are each methyl or phenyl and X is oxygen.

**4.** A process for the preparation of a 4-[(meth)acrylamidomethyl]-pyrazole or -isoxazole of the formula (I) as claimed in any of claims 1 to 3, wherein, in a first step, a 1,3-diketone of the formula (II)

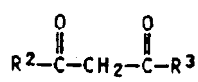

(II)

where $R^2$ and $R^3$ may be identical or different and independently of one another are each alkyl of 1 to 6 carbon atoms or unsubstituted or substituted aryl of 6 to 20 carbon atoms, is condensed with an N-methylol(meth)acrylamide of the formula (III)

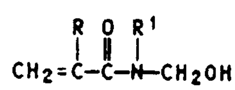

(III)

where R is hydrogen or methyl and $R^1$ is hydrogen, methyl or ethyl, in a strong acid as reaction medium, whereupon the 2-[(meth)acrylamidomethyl]-1,3-diketone of the formula (IV) obtained here

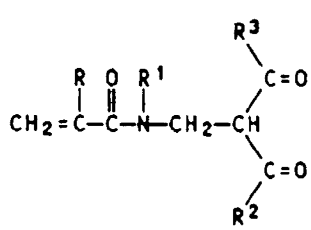

(IV)

where R, $R^1$, $R^2$ and $R^3$ have the meanings stated above for the formulae (II) and (III), is condensed in a second step with (a) a hydrazine or (b) a hydroxylamine.

5. A process as claimed in claim 4, wherein, in the formulae (II), (III) and (IV), R and $R^1$ are each hydrogen and $R^2$ and $R^3$ are each methyl or phenyl.

6. A process as claimed in claim 4 or 5, wherein, in the second step, a 2-[(meth)acrylamidomethyl]-1,3-diketone of the formula (IV) is condensed with a hydrazinium salt or a hydrazine of the formula (V)

$$NH_2\text{-}NH\text{-}R^4 \qquad (V)$$

where $R^4$ is hydrogen or a radical having the meanings of $R^2$ or $R^3$.

7. A process as claimed in claim 4 or 5, wherein, in the second step, a 2-[(meth)acrylamidomethyl]-1,3-diketone of the formula (IV) is condensed with hydroxylamine or a hydroxylammonium salt.

8. Use of a 4-[(meth)acrylamidomethyl]-pyrazole or -isoxazole of the formula (I) as claimed in any of claims 1 to 3 as a polymerizable, in particular photopolymerizable, monomer.

9. Use of a 4-[(meth)acrylamidomethyl]-pyrazole or -isoxazole of the formula (I) as claimed in any of claims 1 to 3 for the preparation of photopolymerizable sealing compounds, surface layers, adhesives or sheet-like recording materials.

10. A homopolymer or copolymer of a 4-[(meth)acrylamidomethyl]-pyrazole of the formula (I) as claimed in claim 1 or 2.

11. A homopolymer or copolymer of a 4-[(meth)acrylamidomethyl]-isoxazole of the formula (I) as claimed in any of claims 1 to 3.

## Revendications

### Revendications pour les Etats contractants suivants : BE, DE, FR, GB, IT, NL, SE

1. 4-[(méth)acrylamidométhyl]-pyrazolés et -isoxazoles de formule générale (1)

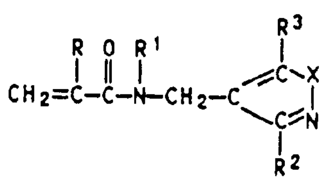

(I),

dans laquelle les symboles R, $R^1$, $R^2$ et $R^3$ et X ont, indépendamment les uns des autres, les significations suivantes :

R : un atome d'hydrogène ou un groupe méthyle ;

$R^1$ : un atome d'hydrogène, un groupe méthyle ou éthyle,

$R^2$, $R^3$ : un groupe alkyle en C1-C6, un groupe aryle non substitué en C6-C20 ou un groupe 2-, 3- ou 4-méthyl-phényle, 2,4-diméthylphényle, 4-tert-butyl, 4-chloro- ou 4-bromo-phényle, 4-phényl-phényle-1 ou 4-(4′-phényl-phényl-1′)-phényle-1, $R^2$ et $R^3$ pouvant avoir des significations identiques ou différentes ;

X : un atome d'oxygène ou un groupe $NR^4$ dans lequel $R^4$ représente un atome d'hydrogène ou l'un des groupes mentionnés en référence à $R^2$ ou $R^3$.

2. 4-[(méth)acrylamidométhyl]-pyrazoles selon la revendication 1, caractérisés en ce que, dans la formule générale I, les symboles R et $R^1$ représentent chacun un atome d'hydrogène, les symboles $R^2$ et $R^3$ représentent chacun un groupe méthyle ou phényle et X représente un groupe $NR^4$ dans lequel $R^4$ est un atome d'hydrogène ou un groupe phényle.

3. 4-[(méth)acrylamidométhyl]-isoxazoles selon la revendication 1, caractérisés en ce que, dans la formule générale I, les symboles R et $R^1$ représentent chacun un atome d'hydrogène, les symboles $R^2$ et $R^3$ re-

présentent chacun un groupe méthyle ou phényle et X un atome d'oxygène.

4. Procédé de préparation des 4-[(méthacrylamidométhyl]-pyrazoles ou -isoxazoles de formule générale I selon l'une des revendications 1 à 3, caractérisé en ce que, dans un premier stade opératoire, on condense une 1,3-dicétone de formule générale II

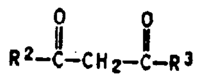

(II),

dans laquelle $R^2$ et $R^3$, ayant des significations identiques ou différents, représentent chacun, indépendamment l'un de l'autre, un groupe alkyle en C1-C6 ou un groupe aryle non substitué ou substitué en C6-C20, avec un N-méthylol-(méth)acrylamide de formule générale III

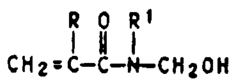

(III),

dans laquelle R représente un atome d'hydrogène ou un groupe méthyle et $R^1$ un atome d'hydrogène, un groupe méthyle ou éthyle, dans un acide fort qui sert de milieu de réaction, la réaction donnant une 2-[(méth)acrylamidométhyl]-1,3-dicétone de formule générale IV

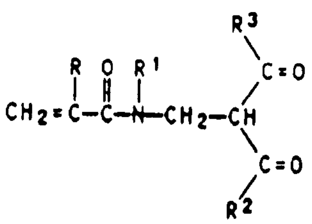

(IV),

dans laquelle R, $R^1$, $R^2$ et $R^3$ ont les significations indiquées ci-dessus en référence aux formules générales II et III, qu'on condense dans un deuxième stade opératoire avec (a) une hydrazine ou (b) une hydroxylamine.

5. Procédé selon la revendication 4, caractérisé en ce que, dans les formules générales II, III et IV, les symboles R et $R^1$ représentent chacun un atome d'hydrogène et les symboles $R^2$ et $R^3$ représentent chacun un groupe méthyle ou phényle.

6. Procédé selon la revendication 4 ou 5, caractérisé en ce que, au deuxième stade opératoire, on condense la 2-[(méth)acrylamidométhyl]-1,3-dicétone de formule générale IV avec un sel d'hydrazinium ou une hydrazine de formule générale V

$$NH^{2}\text{-}NH\text{-}R^{4} \qquad (V),$$

dans laquelle $R^4$ représente un atome d'hydrogène ou a l'une des significations indiquées pour $R^2$ ou $R^3$.

7. Procédé selon la revendication 4 ou 5, caractérisé en ce que, au deuxième stade opératoire, on condense la 2-[(méth)acrylamidométhyl]-1,3-dicétone de formule générale IV avec l'hydroxylamine ou un sel d'hydroxylammonium.

8. Utilisation des 4-[(méth)acrylamidométhyl]-pyrazoles ou -isoxazoles de formule générale I selon l'une des revendications 1 à 3, en tant que monomères polymérisables, en particulier photopolymérisables.

9. Utilisation des 4-[(méth)acrylamidométhyl]-pyrazoles ou -isoxazoles de formule générale I selon l'une des revendications 1 à 3, pour la préparation de masses d'étanchéité, couches superficielles, adhésifs ou matériaux d'enregistrement planiformes photopolymérisables.

10. Homo- et co-polymères des 4-[(méth)acrylamidométhyl]-pyrazoles de formule générale I selon l'une des revendications 1 ou 2.

**11.** Homo- et co-polymères des 4-[(méth)acrylamidométhyl]-isoxazoles de formule générale I selon l'une des revendications 1 ou 3.

**Revendications pour l'Etat contractant suivant : ES**

1. Procédé de préparation des 4-[(méth)acrylamidométhyl]-pyrazoles ou -isoxazoles de formule générale I

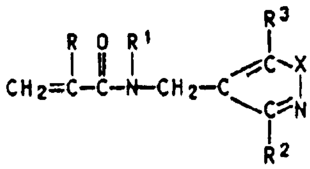

(I),

dans laquelle les symboles R, $R^2$ et $R^3$ et X ont, indépendamment les uns des autres, les significations suivantes :

R : un atome d'hydrogène ou un groupe méthyle ;

$R^1$ : un atome d'hydrogène, un groupe méthyle ou éthyle ;

$R^2$, $R^3$ : un groupe alkyle en C1-C6, un groupe aryle non substitué en C6-C20 ou un groupe 2-, 3- ou 4-méthylphényle, 2,4-diméthylphényle, 4-tert-butyl-, 4-chloro- ou 4-bromo-phényle, 4-phényl-phényle-1 ou 4-(4′-phényl-phényl-1′)-phényle-1, $R^2$ et $R^3$ pouvant avoir des significations identiques ou différentes ;

X : un atome d'hydrogène ou un groupe $NR^4$ dans lequel $R^4$ représente un atome d'hydrogène ou a l'une des significations indiquées pour $R^2$ ou $R^3$ ;

caractérisé en ce que, dans un premier stade opératoire, on condense une 1,3-dicétone de formule générale II

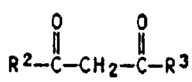

(II),

dans laquelle $R^2$ et $R^3$, ayant des significations identiques ou différentes, représentent chacun, indépendamment l'un de l'autre, un groupe alkyle en C1-C6 ou un groupe aryle non substitué en C6-C20 ou un groupe 2-, 3- ou 4-méthylphényle, 2,4-diméthylphényle, 4-tert-butyl-, 4-chloro- ou 4-bromophényle, 4-phényl-phényl-1 ou 4-(4′-phényl-phényl-1′, avec un N-méthylol-(méth)acrylamide de formule génrale III

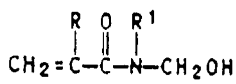

(III),

dans laquelle R représente un atome d'hydrogène ou un groupe méthyle et $R^1$ un atome d'hydrogène, un groupe méthyle ou éthyle, dans un acide fort servant de milieu de réaction, la réaction donnant une 2-[(méth)acrylamidométhyl]-1,3-dicétone de formule générale IV

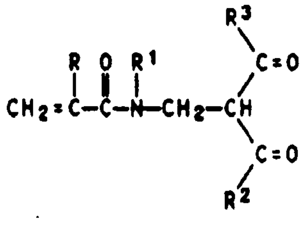

(IV),

dans laquelle les symboles R, $R^1$, $R^2$ et $R^3$ ont les significations indiquées ci-dessus en référence aux formules générales II et III, qu'on condense dans un deuxième stade opératoire avec (a) une hydrazine ou (b) une hydroxylamine.

2. Le procédé selon la revendication 1, caractérisé en ce que, dans les formules générales II, III et IV, les symboles R et $R^1$ représentent chacun un atome d'hydrogène et les symboles $R^2$ et $R^3$ représentent chacun

un groupe méthyle ou phényle.

3. Le procédé selon la revendication 1 ou 2, caractérisé en ce que, dans la formule générale I, les symboles R et $R^1$ représentent chacun un atome d'hydrogène, les symboles $R^2$ et $R^3$ représentent chacun un groupe méthyle ou phényle et X représente un groupe $NR^4$ dans lequel $R^4$ représente un atome d'hydrogène ou un groupe phényle.

4. Le procédé selon la revendication 1 ou 2, caractérisé en ce que, dans la formule générale I, les symboles R et $R^1$ représentent chacun un atome d'hydrogène, les symboles $R^2$ et $R^3$ représentent chacun un groupe méthyle ou phényle et X représente un atome d'oxygène.

5. Le procédé selon l'une des revendications 1 à 3, caractérisé en ce que, au deuxième stade opératoire, on condense la 2-[(méth)acrylamidométhyl]-1,3-dicétone de formule générale IV avec un sel d'hydrazinium ou une hydrazine de formule générale V

$$NH_2\text{-}NH\text{-}R^4 \qquad (V),$$

dans laquelle $R^4$ représente un atome d'hydrogène ou a l'une des significations indiquées pour $R^2$ ou $R^3$.

6. Le procédé selon une des revendications 1, 2 ou 4, caractérisé en ce que, au deuxième stade opératoire, on condense la 2-[(méth)acrylamidométhyl]-1;3-dicétone de formule générale IV avec l'hydroxylamine ou un sel d'hydroxylammonium.

7. Utilisation des 1-[(méth)acrylamidométhyl]-pyrazoles préparés selon l'une des revendications 1 à 3 ou 5 et des 4-[(méth)acrylamidométhyl]-isoxazoles préparés selon l'une des revendications 1, 2, 4 ou 6 de formule générale I, en tant que monomères polymérisables, en particulier photopolymérisables.

8. Utilisation des 4-[(méth)acrylamidométhyl]-pyrazoles et des 4-[(méth)acrylamidométhyl]-isoxazoles de formule générale I préparés selon une des revendicatins 1, 2, 4 ou 6, pour la préparation de masses d'étanchéité, couches superficielles, adhésifs et matériaux d'enregistrement planiformes photopolymérisables.

9 Procédé de préparation de polymères par polymérisation de monomères, caractérisé en ce que l'on utilise en tant que monomère au moins un 4-[(méth)acrylamidométhyl]-pyrazole préparé selon l'une des revendications 1 à 3 ou 5, et/ou au moins un 4[(méth)acrylamidométhyl]-isoxazole préparé selon une des revendications 1, 2, 4 ou 6.

10. Le procédé de préparation de polymères selon la revendication 9, caractérisé en ce que l'on utilise en tant qu'autres monomères au moins un monomère connu en soi.